# EUROPEAN PATENT APPLICATION

(11) **EP 4 233 835 A1**
(43) Date of publication of application: **30.08.2023**
(21) Application number: 21882703.8
(22) Date of filing: 14.10.2021
(51) Int. Cl.: A61K 8/81, A61K 8/06, A61K 8/25, A61K 8/27, A61K 8/29, A61K 8/34, A61K 8/37, A61K 8/60, A61K 8/73, A61Q 17/04

(54) **OIL-IN-WATER TYPE COMPOSITION**

(30) Priority: 21.10.2020 JP 2020176847
(71) Applicant: Shiseido Company, Ltd., Chuo-ku Tokyo 104-0061 (JP)
(72) Inventor: ENOMOTO Ayumu, Tokyo 104-0061 (JP); NISHIDA Keita, Tokyo 104-0061 (JP)
(74) Representative: Ter Meer Steinmeister & Partner
(86) International application number: PCT/JP2021/038074
(87) International publication number: WO 2022/085562

(57) **Abstract**

A water-in-oil composition contains (A) from 0.02% by mass to 0.25% by mass of a water-soluble polymer, (B) from 0.2% by mass to 2.5% by mass of at least one component selected from the group consisting of sugars and sugar alcohols, which are in a solid form under atmospheric pressure at 25 °C, (C) water, (D) a first powder having a hydrophobic particle surface, (E) a second powder having a hydrophilic particle surface, and (F) an oily component. Component (A) is at least one selected from the group consisting of polyacrylic acid, poly(2-acrylamido-2-methylpropane sulfonic acid), and salts thereof. Component (E) has a linseed oil absorption amount at 25°C of 170 ml or less per 100 g.

## Description

### Cross Reference to Related Applications

The present application is based upon and claims the benefit of priority from Japanese Patent Application No. 2020-176847 filed on October 21, 2020, the entire contents of which are incorporated herein by reference.

### Technical Field

The present disclosure relates to a water-in-oil composition.

### Background Art

Water-in-oil compositions are used, for example, for external-use skin preparations, such as sun-screen cosmetics (see, for example, Patent Literature 1). Patent Literature 1 discloses a water-in-oil sun-screen cosmetic that contains a volatile component, an organomodified clay mineral and a spherical resin powder, and that does not contain a UV absorber.

### Citation List

### Patent Literature

Patent Literature 1: Japanese Unexamined Patent Publication No. H09-255544

### Summary of Invention

### Technical Problem

The following analysis can be made from the perspective of the present disclosure.

Some sun-screen cosmetics contain powder as a UV scattering agent. In order to improve UV protection effects, such sun-screen cosmetics may contain large amounts of UV scattering agent. Unfortunately, when a water-in-oil composition containing a large amount of hydrophobic powder, such as a UV scattering agent, is applied to the skin, the hydrophobic powder may aggregate on the skin at the time of application, and the user may perceive squeaky feeling caused by the hydrophobic powder. To suppress such squeaky feeling, there are cases where a highly oil-absorbent powder such as resin powder is added, as in the water-in-oil sun-screen cosmetic disclosed in Patent Literature 1. However, the presence of such a highly oil-absorbent powder in a water-in-oil composition-whose aqueous phase contains thickeners and/or moisturizers for achieving a moist feel upon use-may impair the spreadability at the time of application.

Hence, there has been a demand for a water-in-oil composition that spreads easily/lightly upon application and can offer a moist feel after application, without giving rise to powder-induced squeaky feeling at the time of application to the skin.

### Solution to Problem

According to a first aspect of the present disclosure, a water-in-oil composition is provided, the composition comprising (A) from 0.02% by mass to 0.25% by mass of a water-soluble polymer, (B) from 0.2% by mass to 2.5% by mass of at least one component selected from the group consisting of sugars and sugar alcohols, the component being in a solid form under atmospheric pressure at 25 °C, (C) water, (D) a first powder having a hydrophobic particle surface, (E) a second powder having a hydrophilic particle surface, and (F) an oily component. The component (A) is at least one selected from the group consisting of polyacrylic acid, poly(2-acrylamido-2-methylpropane sulfonic acid), and salts thereof. The component (E) has a linseed oil absorption amount at 25°C of 170 ml or less per 100 g.

### Advantageous Effects of Invention

In the water-in-oil composition of the present disclosure, squeaky feeling at the time of application to the skin, which is caused by hydrophobic powder, is suppressed. Further, the water-in-oil composition of the present disclosure can be applied with light spreadability. Thus, the user can apply the water-in-oil composition with a comfortable feeling. The user can also feel moistness on the skin when the water-in-oil composition of the present disclosure is applied to the skin.

### Description of Embodiments

Preferred modes according to the aforementioned aspects will be described below.

According to a prefered mode of the above first aspect, a content of the component (D) is from 2% by mass to 20% by mass relative to the mass of the composition.

According to a prefered mode of the above first aspect, a content of the component (E) is from 1% by mass to 5% by mass relative to the mass of the composition.

According to a prefered mode of the above first aspect, the component (B) is at least one selected from the group consisting of trehalose, xylitol, and erythritol.

According to a prefered mode of the above first aspect, the component (D) is at least one selected from the group consisting of hydrophobized titanium dioxide and hydrophobized zinc oxide.

According to a prefered mode of the above first aspect, the component (E) is at least one selected from the group consisting of silica, corn starch, and cellulose.

According to a prefered mode of the above first aspect, the component (F) contains a liquid-state oily component and a UV absorber.

According to a prefered mode of the above first aspect, the component (A) has a weight-average molecular weight of from 500,000 to 8,000,000.

According to a prefered mode of the above first aspect, a content of a resinous powder having an average particle size of from 1 µm to 20 µm is 0.1% by mass or less relative to the mass of the composition.

According to a prefered mode of the above first aspect, the water-in-oil composition is for use in an external-use skin preparation.

In the following description, POE is an abbreviation of polyoxyethylene, and POP is an abbreviation of polyoxypropylene. The number in parentheses after POE or POP indicates the average number of moles of POE groups or POP groups added in the compound in question.

In the present disclosure, "substantial amount" refers to an amount capable of bringing about the actions/effects achieved by the addition of the compound in question.

### First Embodiment:

A water-in-oil composition according to a first embodiment of the present disclosure will be described. The water-in-oil composition according to the first embodiment includes (A) a water-soluble polymer, (B) at least one component selected from the group consisting of sugars and sugar alcohols, the component being in a solid form under atmospheric pressure at 25°C, (C) water, (D) a first powder, (E) a second powder, and (F) an oily component. In the water-in-oil composition, it is thought that at least a portion of the component (A) and at least a portion of the component (B) are entrapped in the aqueous phase.

### (A) Water-Soluble Polymer:

The component (A) is at least one selected from the group consisting of polyacrylic acid, poly(2-acrylamido-2-methylpropane sulfonic acid), and salts thereof. Hereinbelow, polyacrylic acid and/or salts thereof are referred to as "polyacrylic acid salt" or "polyacrylate".

Types of salts of the component (A) may include, for example: alkali metal salts (e.g., sodium salts, potassium salts, magnesium salts, calcium salts, etc.); organic amine salts (e.g., monoethanolamine salts, diethanolamine salts, triethanolamine salts, triisopropanolamine salts, etc.); and salts of basic nitrogen-containing compounds such as 2-amino-2-methyl-1-propanol, 2-amino-2-methyl-1, 3-propane diol, 2-amino-2-hydroxymethyl-1, 3-propane diol, L-arginine, L-lysine, L-alkyltaurine, etc. Among the above, monovalent alkali metal salts and organic amine salts are preferred, and sodium salts, potassium salts and triethanolamine salts are more preferred, and sodium salts are even more preferred.

The component (A) may have a weight-average molecular weight of, for example, 500,000 or higher, 1,000,000 or higher, or 2,000,000 or higher. The component (A) may have a weight-average molecular weight of, for example, 20,000,000 or lower, 15,000,000 or lower, 10,000,000 or lower, 8,000,000 or lower, or 5,000,000 or lower.

The component (A) may be a water-soluble polymer with a controlled molecular weight (referred to hereinafter as "molecular-weight-controlled water-soluble polymer"). The weight-average molecular weight of the molecular-weight-controlled water-soluble polymer may be 500,000 or higher. The weight-average molecular weight of the molecular-weight-controlled water-soluble polymer may be 8,000,000 or lower. It is preferred that the molecular-weight-controlled water-soluble polymer is a polymer whose molecular weight (the distribution thereof) is controlled. As regards the molecular-weight-controlled water-soluble polymer, it is preferred that the content of polymers having a molecular weight of 10,000,000 or higher is 10% by mass or less relative to the total amount of the water-soluble polymer. It is preferred that a main polymer chain in the molecular-weight-controlled water-soluble polymer is linear. It is preferred that the molecular-weight distribution (i.e., weight-average molecular weight/number-average molecular weight) of the molecular-weight-controlled water-soluble polymer is preferably 2 or lower, more preferably 1.8 or lower. It is thought that, with a molecular-weight-controlled polymer, it is possible to further improve the distribution uniformity of the various components when the water-in-oil composition is applied to the skin. As regards the molecular-weight-controlled water-soluble polymer, the disclosure of WO2015/052804 is incorporated herein by reference.

The molecular-weight-controlled water-soluble polymer can be synthesized by any known living polymerization method. Examples of living polymerization may include living anionic polymerization, living cationic polymerization, and living radical polymerization (precision radical polymerization or controlled radical polymerization). Examples of living radical polymerization may include: (radical) polymerization mediated by nitroxide, or nitroxide-mediated (radical) polymerization (NLRP); atom transfer radical polymerization (ATRP); and reversible addition-fragmentation chain transfer (RAFT) polymerization. Examples of atom transfer radical polymerization (ATRP) may include: electron transfer generated activator ATRP, or activators generated by electron transfer ATRP (AGET ATRP); electron transfer regenerated activator ATRP, or activators regenerated by electron transfer ATRP (ARGET ATRP); initiators to continuously regenerate active species ATRP, or initiators for continuous activator regeneration ATRP (ICAR ATRP); and reverse ATRP (Reverse ATRP). Examples of derivative technology of the reversible addition-fragmentation chain transfer (RAFT) polymerization may include: living radical polymerization in which organic tellurium is the growing end, or organic tellurium-mediated living radical polymerization (TERP); antimony-mediated living radical polymerization (SBRP); and bismuth-mediated living radical polymerization (BIRP). Examples of other living radical polymerizations may include iodine transfer radical polymerization (IRP) and cobalt-mediated radical polymerization (CMRP). Among the above, it is preferred to employ the reversible addition-fragmentation chain transfer polymerization method (RAFT polymerization method) because this technology enables synthesis of polymer compounds with a narrow molecular weight distribution. Preferred examples of chain transfer agents include dithio-type and trithio-type agents. It is preferred to employ a polymerization initiator having a chemical structure similar to the chain transfer agent, and azo-type initiators are preferred. The polymerization solvent is not particularly limited; it is possible to select, as appropriate, a solvent having a high capability of dissolving monomers and polymers. Preferably, the polymerization time is from a few hours to around 100 hours.

The molecular weight of the component (A) can be measured according to any known method; for example, weight-average molecular weight can be measured by e.g. light scattering, ultracentrifugation, or chromatography; number-average molecular weight can be measured by e.g., the osmotic pressure method or chromatography. Among the above, chromatography is preferable in terms that weight-average molecular weight, number-average molecular weight, and molecular weight distribution can be obtained easily with a small amount of sample, and gel permeation chromatography (abbreviated as "GPC" hereinbelow) is preferred. Molecular weight distribution can be expressed as a value found by dividing the weight-average molecular weight obtained by GPC analysis by the number-average molecular weight.

The content of the component (A) relative to the mass of the water-in-oil composition is preferably 0.02% by mass or greater. The content of the component (A) relative to the mass of the water-in-oil composition may be 0.05% by mass or greater, 0.1% by mass or greater, 0.12% by mass or greater, or 0.15% by mass or greater. If the content of the component (A) is less than 0.02% by mass, it is not possible to improve the feel upon use when the composition is applied to the skin. The content of the component (A) relative to the mass of the water-in-oil composition is preferably 0.25% by mass or less. The content of the component (A) relative to the mass of the water-in-oil composition may be 0.22% by mass or less, 0.2% by mass or less, 0.15% by mass or less, or 0.1% by mass or less. If the content of the component (A) exceeds 0.25% by mass, the feel upon use when the composition is applied to the skin may deteriorate.

### (B) Sugar and/or Sugar Alcohol:

The sugar and sugar alcohol are in a solid form under atmospheric pressure at 25°C. In the present disclosure, "solid form" refers to a form/state capable of maintaining a solid form under atmospheric pressure at 25°C. In the present disclosure, "liquid state" refers to a form/state having flowability (i.e., is a liquid) under atmospheric pressure at 25°C.

Examples of sugars may include trehalose etc.

Examples of sugar alcohols may include xylitol, erythritol, etc.

The content of the component (B) relative to the mass of the water-in-oil composition is preferably 0.2% by mass or greater. The content of the component (B) relative to the mass of the water-in-oil composition may be 0.5% by mass or greater, 1% by mass or greater, 1.2% by mass or greater, or 1.5% by mass or greater. If the content of the component (B) is less than 0.2% by mass, it is not possible to improve the feel upon use when the composition is applied to the skin. The content of the component (B) relative to the mass of the water-in-oil composition is preferably 2.5% by mass or less. The content of the component (B) relative to the mass of the water-in-oil composition may be 2.2% by mass or less, 2% by mass or less, 1.5% by mass or less, or 1% by mass or less. If the content of the component (B) exceeds 2.5% by mass, the feel upon use when the composition is applied to the skin may deteriorate.

### (C) Water:

Examples of water may include water used for cosmetics, quasi-pharmaceutical products, or the like, and usable examples may include purified water, ion-exchanged water, or tap water.

The content of the component (C) relative to the mass of the water-in-oil composition may be 10% by mass or greater, 15% by mass or greater, 20% by mass or greater, 25% by mass or greater, 30% by mass or greater, 35% by mass or greater, 40% by mass or greater, or 45% by mass or greater. The content of the component (C) relative to the mass of the water-in-oil composition may be 60% by mass or less, 55% by mass or less, 50% by mass or less, 45% by mass or less, 40% by mass or less, 35% by mass or less, 30% by mass or less, 25% by mass or less, or 20% by mass or less.

### (D) First Powder:

The first powder is a powder having a hydrophobic particle surface. The first powder may include a powder whose particle surface has been hydrophobized. It is thought that the first powder is present in the oil phase (outer phase) of the water-in-oil composition. An example of a hydrophobizing method may be to coat the particles of the powder with silicone resin, a fatty acid, etc. The first powder may be, for example, a hydrophobized metal oxide powder. The metal oxide may be, for example, a powder that acts as a UV scattering agent. Examples of the metal oxide may include zinc oxide, titanium dioxide, etc.

The average particle size of the primary particles of the first powder is preferably 5 nm or greater. The average particle size of the primary particles of the first powder is preferably 50 nm or less.

The average particle size of the primary particles of the first powder can be measured by employing dynamic light scattering.

The linseed oil absorption amount of the first powder per 100 g of the first powder at 25°C may be 170 ml or less. The linseed oil absorption amount can be measured according to JIS K5101-13-2 (Boiled Linseed Oil Method).

The content of the component (D) relative to the mass of the water-in-oil composition is preferably 2% by mass or greater, more preferably 4% by mass or greater. The content of the component (D) relative to the mass of the water-in-oil composition may be 6% by mass or greater, 8% by mass or greater, 10% by mass or greater, or 12% by mass or greater. In cases where the component (D) is a UV scattering agent, if the content of the component (D) is less than 2% by mass, it is not possible to obtain a sufficient UV protection effect. The content of the component (D) relative to the mass of the water-in-oil composition is preferably 20% by mass or less. The content of the component (D) relative to the mass of the water-in-oil composition may be 18% by mass or less, 16% by mass or less, 14% by mass or less, 12% by mass or less, or 10% by mass or less. If the content of the component (D) exceeds 20% by mass, a squeaky-powdery feel may arise when the composition is applied to the skin.

In the present disclosure, "squeaky feeling" refers to a feel of lack of smoothness at the time of application, which is perceived when the composition is being applied to the skin. A user may start feeling squeakiness after applying the composition to the skin, and squeaky feeling may further increase over time. Squeaky feeling ascribable to powder is referred to as "squeaky-powdery feel".

### (E) Second Powder:

The second powder is a powder having a hydrophilic particle surface. The second powder may include a powder whose particle surface has been hydrophilized.

The linseed oil absorption amount of the second powder per 100 g of the second powder at 25°C may be 170 ml or less. The linseed oil absorption amount can be measured according to JIS K5101-13-2 (Boiled Linseed Oil Method).

For the second powder, it is possible to use, for example, silica, corn starch, cellulose, etc.

The average particle size of the primary particles of the second powder is preferably 0.5 nm or greater. The average particle size of the primary particles of the second powder is preferably 50 nm or less.

The average particle size of the primary particles of the second powder can be measured by employing dynamic light scattering.

The content of the component (E) relative to the mass of the water-in-oil composition is preferably 1% by mass or greater. The content of the component (E) relative to the mass of the water-in-oil composition may be 1.5% by mass or greater. If the content of the component (E) is less than 1% by mass, the feel upon use cannot be improved. The content of the component (E) relative to the mass of the water-in-oil composition is preferably 5% by mass or less. The content of the component (E) relative to the mass of the water-in-oil composition may be 4% by mass or less, 3% by mass or less, or 2.5% by mass or less. If the content of the component (E) exceeds 5% by mass, the dispersibility of the second powder will deteriorate.

### (F) Oily Component:

It is preferred that the oily component includes a liquid-state oily component. It is preferred that the liquid-state oily component includes at least one selected from the group consisting of liquid-state hydrocarbon oils, liquid-state ester oils, liquid-state higher alcohols, and liquid-state silicone oils. The oily component may include an oil-soluble UV absorber. In cases where the oily component includes an oil-soluble UV absorber, it is preferred that the liquid-state oily component is miscible with the oil-soluble UV absorber.

Examples of hydrocarbon oils may include liquid paraffin, ozokerite, squalane, pristane, paraffin, ceresin, squalene, isododecane, isohexadecane, hydrogenated polydecene, mineral oils, etc.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber, cyclopentasiloxane and the like.

Examples of oil-soluble UV absorbers may include: benzoic acid-based UV absorbers (e.g., para-aminobenzoic acid (abbreviated as PABA hereinbelow), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid-based UV absorbers (e.g., homomenthyl-N-acetylanthranilate, etc.); salicylic acid-based UV absorbers (e.g., amyl salicylate, menthyl salicylate, homomenthyl salicylate, octyl salicylate, phenyl salicylate, benzyl salicylate, p-isopropanol phenyl salicylate, homosalate, etc.); cinnamic acid-based UV absorbers (e.g., octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, ethylhexyl methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, glyceryl mono-2-ethylhexanoyl-di-para-methoxycinnamate, etc.); 3-(4'-methylbenzylidene)-d,l-camphor, 3-benzylidene-d,l-camphor; 2-phenyl-5-methylbenzoxazole; 2,2'-hydroxy-5-methylphenyl benzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenyl acrylate (octocrylene); 2,4-bis-{[4-(2-ethylhexyloxy)-2-hydroxy]-phenyl}-6-(4-methoxyphenyl)-(1,3,5)-triazine; and benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4' -phenyl-benzophenone-2-carboxylate, 2-hydroxy-4-n-octoxybenzophenone, 4-hydroxy-3-carboxybenzophenone, etc.).

The content of the component (F) relative to the mass of the water-in-oil composition may be 20% by mass or greater, 25% by mass or greater, 30% by mass or greater, or 35% by mass or greater. The content of the component (F) relative to the mass of the water-in-oil composition may be 60% by mass or less, 55% by mass or less, 50% by mass or less, 45% by mass or less, 40% by mass or less, 35% by mass or less, or 30% by mass or less.

### (G) Surfactant:

The water-in-oil composition according to the first embodiment may further include a surfactant. The surfactant is not limited so long as it can emulsify the aqueous phase. Examples of the surfactant may include anionic surfactants, cationic surfactants, amphoteric surfactants, hydrophilic nonionic surfactants, and lipopholic nonionic surfactants. In cases where the water-in-oil composition is not to be rinsed off after application to the skin, it is preferred that the surfactant is a nonionic surfactant.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

The content of the surfactant to the mass of the water-in-oil composition may be, for example, 0.2% by mass or greater, 0.5% by mass or greater, or 1% by mass or greater. The content of the surfactant to the mass of the water-in-oil composition may be, for example, 7% by mass or less, 5% by mass or less, 3% by mass or less, or 2% by mass or less.

### (H) Others:

If necessary, the water-in-oil composition of the present disclosure may contain other components as appropriate, such as alcohols, powders other than the above, oily components other than the above, thickeners, moisturizers, film-forming agents, water-soluble UV absorbers, metal ion sequestering agents, amino acids, organic amines, polymer emulsions, pH adjusters, skin nutrients, vitamins, antioxidants, antioxidant aids, perfumes, etc., in amounts that do not inhibit the effects of the present disclosure.

Examples of the water-soluble alcohol may include at least one selected from, for example, lower alcohols, polyols, polyol polymers, divalent alcohol alkyl ethers, divalent alcohol alkyl ethers, divalent alcohol ether esters, glycerin monoalkyl ethers, sugar alcohols, monosaccharides, oligosaccharides, polysaccharides, and derivatives thereof.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3 -butylene glycol, pentamethylene glycol, 2-butene-1,4-diol, hexylene glycol, octylene glycol, 1,2-hexanediol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxy acetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparan sulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium dioxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium dioxide coated mica, titanium dioxide coated bismuth oxychloride, titanium dioxide coated talc, colored titanium dioxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No. 104, Red No. 106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc); wax powder (such as carnauba wax powder, etc), starch powder (such as cornstarch powder, rice starch powder, etc) and the like.

Examples of the waxes that may be used may include beeswax, candelilla wax, cotton wax, carnauba wax, bayberry wax, insect wax, montan wax, bran wax, kapok wax, sugarcane wax, hexyl laurate, jojoba wax, shellac wax, POE lanolin alcohol ether, POE cholesterol ether, POE hydrogenated lanolin alcohol ether, liquid paraffin, ozocerite, squalane, pristane, paraffin, ceresin. squalene, microcrystalline wax, Físcher-Trópsch wax, for example, and the like.

Examples of the liquid oil that may be used may include avocado oil, camellia oil, macadamia nut oil, corn oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, hydrogenated coconut oil, palm oil, palm kernel oil, Japan wax kernel oil, hardened oil, Japan wax, hydrogenated caster oil, and the like.

Examples of the higher fatty acid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride, taurate-based synthetic polymers, and acrylate-based synthetic polymers, and the like.

Examples of the moisturizers may include polyethylene glycol, propylene glycol, glycerin, 1,3-butylene glycol, xylitol, sorbitol, maltitol, diglycerin (EO)PO adduct, chestnut rose extract, yarrow extract, melilot extract, and the like.

Examples of the film-forming agent may include polymeric silicone, silicone resin, trimethylsiloxysilicate, and the like.

Examples of water-soluble UV absorbers may include: benzophenone-based UV absorbers (such as 2-hydroxy-4-methoxybenzophenone-5-sulfate, etc.); benzylidene camphor-based UV absorbers (such as benzylidene camphor sulfonic acid, terephthalylidene dicamphor sulfonic acid, etc.); phenylbenzimidazole-based UV absorbers (such as phenylbenzimidazole sulfonic acid, etc.), and the like

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B 1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure may further contain, as appropriate: caffeine, tannin, verapamil, tranexamic acid and derivatives thereof; various herbal medicine extracts such as licorice, pseudocydonia sinensis, pyrola japonica, etc.; drugs such as tocopherol acetate, glycyrrhetinic acid, glycyrrhizinic acid and derivatives or salts thereof; skin whitening agents such as vitamin C, magnesium ascorbyl phosphate, ascorbic acid glucoside, arbutin, kojic acid, etc.; amino acids such as arginine, lysine, etc., and derivatives thereof.

### Resinous Powder:

In the water-in-oil composition of the present disclosure, it is preferred that the content of resinous powder having an average particle size of from 1 µm to 20 µm relative to the mass of the composition is preferably 0.1% by mass or less, and more preferably, the composition includes no substantial amount of resinous powder (0% by mass). If the content of resinous powder exceeds 0.1% by mass, spreadability at the time of application becomes heavy. A resinous powder is a powder whose linseed oil absorption amount per 100 g of resinous powder exceeds 170 ml.

### Manufacturing Method:

A method for manufacturing the water-in-oil composition of the present disclosure will be described. The water-in-oil composition of the present disclosure can be manufactured according to a known method. For example, the manufacturing method may involve: a step of preparing an aqueous phase by mixing the component (A), the component (B), the component (C), and the component (E); and a step of emulsifying the aqueous phase in an oil phase including the component (D) and the component (F) by using a surfactant.

There may be cases where it is difficult, or utterly impractical, to directly define the phase structure, emulsification form, etc., of the water-in-oil composition of the present disclosure based on the compositional makeup etc. thereof. In such circumstances, it should be permissible to define the water-in-oil composition of the present disclosure according to methods for producing the same.

In the water-in-oil composition of the present disclosure, by adding, to the aqueous phase, a water-soluble polymer acting as a thickener and a sugar/sugar alcohol acting as a moisturizer, the user can feel moistness after applying the composition to the skin. Further, the water-soluble polymer and the sugar/sugar alcohol suppress aggregation of hydrophobic powder on the skin at the time of application onto the skin, without the need to add a highly oil-absorbent powder. Thus, squeaky feeling at the time of application can be suppressed, and the user can obtain a pleasant feeling upon application. Furthermore, since no highly oil-absorbent powder is included, it is possible to achieve an easily-spreadable feeling upon application.

The water-in-oil composition of the present disclosure is applicable, for example, to external-use skin preparations. The water-in-oil composition of the present disclosure is applicable, for example, to base makeup, veil makeup, makeup cosmetics, antiperspirants, deodorants, sun-screen cosmetics, skin-care agents, cleansers, etc.

### Examples

The water-in-oil composition of the present disclosure will be described below by way of examples. The water-in-oil composition of the present disclosure is, however, not limited to the following examples. The content by percentage of each of the components shown in the Tables is in terms of percent by mass (mass%).

### Test Examples 1 to 23:

Water-in-oil external-use skin preparation compositions (sun-screen cosmetics) described below were prepared. The moistness of each composition after application to the skin was evaluated. The evaluation methods and evaluation criteria for each of the test items are described below.

### Moistness after Application:

Ten expert panelists applied each sample to their arm and evaluated whether they felt moistness. Also, the presence/absence of squeaky feeling at the time of application and the lightness of spreadability of each sample were evaluated. Evaluation was conducted by comparing each sample with the composition of Test Example 1 employing vinyl dimethicone/methicone silsesquioxane crosspolymer powder, which is a highly oil-absorbent powder. Each evaluation item was rated as follows according to the number of panelists.
A: Seven or more panelists felt that the sample was comparable to or better than the composition of Test Example 1.
B: Four to six panelists felt that the sample was comparable to or better than the composition of Test Example 1.
C: Three or fewer panelists felt that the sample was comparable to or better than the composition of Test Example 1.

### Test Examples 1 to 9:

In Test Examples 1 to 9, differences in the feel upon use were tested depending on whether or not (A) polyacrylic acid and/or a salt thereof, and/or (B) a sugar and/or a sugar alcohol was/were included. Tables 1 and 2 show the compositional makeup and evaluation for each of the compositions.

Test Examples 4 to 7, which did not include at least one of the component (A) and the component (B), and Test Examples 8 and 9, which did not include any second powder (E) having low oil absorbency, had poor ratings for all evaluation items. In contrast, as regards Test Examples 2 and 3, which included the components (A), (B), and (E), many panelists felt moistness. Also, many panelists did not feel squeaky feeling at the time of application, and felt light spreadability.

**[Table 1]**

| Test Example | | 1 | 2 | 3 | 4 | 5 |
|---|---|---|---|---|---|---|
| (A) Sodium polyacrylate ^{*1} | | - | 0.05 | 0.05 | - | - |
| (B) Trehalose | | - | 0.5 | 0.5 | - | - |
| (C) Ethanol | | 10 | 10 | 10 | 10 | 10 |
| (C) Water | | Balance | Balance | Balance | Balance | Balance |
| (D) Hydrophobized (silicone resin-coated) titanium dioxide ^{*2} | | 11 | 11 | 11 | 11 | 11 |
| (E) Silica ^{*2} | | - | 2 | - | - | 2 |
| (E) Corn starch ^{*3} | | - | - | 2 | - | - |
| (E') Vinyl dimethicone/methicone silsesquioxane crosspolymer ^{*4} | | 2 | - | - | - | - |
| (F) Dimethicone | | 25 | 25 | 25 | 25 | 25 |
| (F) Octocrylene | | 3 | 3 | 3 | 3 | 3 |
| (F) Ethylhexyl methoxycinnamate | | 7 | 7 | 7 | 7 | 7 |
| Disteardimonium hectorite | | 0.5 | 0.5 | 0.5 | 0.5 | 0.5 |
| PEG-9 polydimethylsiloxyethyl dimethicone | | 3 | 3 | 3 | 3 | 3 |
| Total | | 100 | 100 | 100 | 100 | 100 |
| Evaluation | Moistness | Reference | A | A | C | C |
| | Without squeaky feeling | Reference | A | A | C | B |
| | Lightness of spreadability | Reference | A | A | C | C |
| *1: Weight-average molecular weight: 2,000,000; content of polymers having weight-average molecular weight of 10,000,000 or higher relative to mass of sodium polyacrylate: 10% by mass or less | | | | | | |
| *2: Linseed oil absorption amount per 100 g of silica at 25°C: 160 ml | | | | | | |
| *3: Linseed oil absorption amount per 100 g of corn starch at 25°C: 25 ml | | | | | | |
| *4: Linseed oil absorption amount per 100 g of vinyl dimethicone/methicone silsesquioxane crosspolymer at 25°C: 225 ml | | | | | | |

**[Table 2]**

| Test Example | | 6 | 7 | 8 | 9 |
|---|---|---|---|---|---|
| (A) Sodium polyacrylate ^{*1} | | - | 0.05 | 0.05 | 0.05 |
| (B) Trehalose | | 0.5 | - | 0.5 | 0.5 |
| (C) Ethanol | | 10 | 10 | 10 | 10 |
| (C) Water | | Balance | Balance | Balance | Balance |
| (D) Hydrophobized (silicone resin-coated) titanium dioxide | | 11 | 11 | 11 | 11 |
| (E) Silica ^{*2} | | 2 | 2 | - | - |
| (E) Corn starch ^{*3} | | | | | |
| (E') Vinyl dimethicone/methicone silsesquioxane crosspolymer ^{*4} | | - | - | 2 | - |
| (F) Dimethicone | | 25 | 25 | 25 | 25 |
| (F) Octocrylene | | 3 | 3 | 3 | 3 |
| (F) Ethylhexyl methoxycinnamate | | 7 | 7 | 7 | 7 |
| Disteardimonium hectorite | | 0.5 | 0.5 | 0.5 | 0.5 |
| PEG-9 polydimethylsiloxyethyl dimethicone | | 3 | 3 | 3 | 3 |
| Total | | 100 | 100 | 100 | 100 |
| Evaluation | Moistness | C | B | B | B |
| | Without squeaky feeling | B | B | B | C |
| | Lightness of spreadability | C | B | C | B |

### Test Examples 10 to 12:

In Test Examples 10 to 12, tests were conducted by adding other water-soluble polymers (thickeners) instead of the component (A). Table 3 shows the compositional makeup and evaluation for each of the compositions.

Water-soluble polymers different from polyacrylic acid salts could not improve the rating for any of the evaluation items.

**[Table 3]**

| Test Example | | 10 | 11 | 12 |
|---|---|---|---|---|
| (A') Carboxyvinyl polymer | | 0.05 | - | - |
| (A') Agar | | - | 0.05 | - |
| (A') Succinoglycan | | - | - | 0.05 |
| (B) Trehalose | | 0.5 | 0.5 | 0.5 |
| (C) Ethanol | | 10 | 10 | 10 |
| (C) Water | | Balance | Balance | Balance |
| (D) Hydrophobized (silicone resin-coated) titanium dioxide | | 11 | 11 | 11 |
| (E) Silica ^{*2} | | 2 | 2 | 2 |
| (F) Dimethicone | | 25 | 25 | 25 |
| (F) Octocrylene | | 3 | 3 | 3 |
| (F) Ethylhexyl methoxycinnamate | | 7 | 7 | 7 |
| Disteardimonium hectorite | | 0.5 | 0.5 | 0.5 |
| PEG-9 polydimethylsiloxyethyl dimethicone | | 3 | 3 | 3 |
| Total | | 100 | 100 | 100 |
| Evaluation | Moistness | C | B | B |
| | Without squeaky feeling | B | C | C |
| | Lightness of spreadability | B | C | C |

### Test Examples 13 to 17:

In Test Examples 13 to 17, tests were conducted by adding other moisturizer components instead of the component (B). Table 4 shows the compositional makeup and evaluation for each of the compositions.

Test Examples 13 and 14, which included a sugar alcohol as the component (B), were able to improve the ratings for all the evaluation items. In contrast, Test Examples 15 to 17, which included moisturizer components, other than sugars and sugar alcohols, instead of the component (B) had lower ratings for all the evaluation items.

**[Table 4]**

| Test Example | | 13 | 14 | 15 | 16 | 17 |
|---|---|---|---|---|---|---|
| (A) Sodium polyacrylate ^{*1} | | 0.05 | 0.05 | 0.05 | 0.05 | 0.05 |
| (B) Xylitol | | 0.5 | - | - | - | - |
| (B) Erythritol | | - | 0.5 | - | - | - |
| (B') Glycerin | | - | - | 5 | - | - |
| (B') 1,3-Butylene glycol | | - | - | - | 5 | - |
| (B') Phytosteryl macadamiate | | - | - | - | - | 1 |
| (C) Ethanol | | 10 | 10 | 10 | 10 | 10 |
| (C) Water | | Balance | Balance | Balance | Balance | Balance |
| (D) Hydrophobized (silicone resin-coated) titanium dioxide | | 11 | 11 | 11 | 11 | 11 |
| (E) Silica ^{*2} | | 2 | 2 | 2 | 2 | 2 |
| (F) Dimethicone | | 25 | 25 | 25 | 25 | 25 |
| (F) Octocrylene | | 3 | 3 | 3 | 3 | 3 |
| (F) Ethylhexyl methoxycinnamate | | 7 | 7 | 7 | 7 | 7 |
| Disteardimonium hectorite | | 0.5 | 0.5 | 0.5 | 0.5 | 0.5 |
| PEG-9 polydimethylsiloxyethyl dimethicone | | 3 | 3 | 3 | 3 | 3 |
| Total | | 100 | 100 | 100 | 100 | 100 |
| Evaluation | Moistness | A | A | C | C | B |
| | Without squeaky feeling | A | A | B | B | B |
| | Lightness of spreadability | A | A | C | B | C |

### Test Examples 18 to 21:

In Test Examples 18 to 21, tests were conducted by changing the content by percentage of the components (A) and (B). Table 5 shows the compositional makeup and evaluation for each of the compositions.

Comparing Test Example 18 and Test Example 19, it is conceivable that the content of the component (A) relative to the mass of the composition is preferably 0.25% by mass or less. Comparing Test Example 20 and Test Example 21, it is conceivable that the content of the component (B) relative to the mass of the composition is preferably 2.5% by mass or less.

**[Table 5]**

| Test Example | | 18 | 19 | 20 | 21 |
|---|---|---|---|---|---|
| (A) Sodium polyacrylate ^{*1} | | 0.2 | 0.3 | 0.05 | 0.05 |
| (B) Trehalose | | 0.5 | 0.5 | 2 | 3 |
| (C) Ethanol | | 10 | 10 | 10 | 10 |
| (C) Water | | Balance | Balance | Balance | Balance |
| (D) Hydrophobized (silicone resin-coated) titanium dioxide | | 11 | 11 | 11 | 11 |
| (E) Silica ^{*2} | | 2 | 2 | 2 | 2 |
| (F) Dimethicone | | 25 | 25 | 25 | 25 |
| (F) Octocrylene | | 3 | 3 | 3 | 3 |
| (F) Ethylhexyl methoxycinnamate | | 7 | 7 | 7 | 7 |
| Disteardimonium hectorite | | 0.5 | 0.5 | 0.5 | 0.5 |
| PEG-9 polydimethylsiloxyethyl dimethicone | | 3 | 3 | 3 | 3 |
| Total | | 100 | 100 | 100 | 100 |
| Evaluation | Moistness | A | B | A | B |
| | Without squeaky feeling | A | A | A | A |
| | Lightness of spreadability | A | B | A | B |

### Test Examples 22 and 23:

In Test Examples 22 and 23, tests were conducted by changing the molecular weight and the type of the component (A). Table 6 shows the compositional makeup and evaluation for each of the compositions.

Both Test Example 22, which used sodium polyacrylate having a molecular weight different from the aforementioned test example, and Test Example 23, which used poly(2-acrylamido-2-methylpropane sulfonic acid), were able to obtain favorable ratings.

**[Table 6]**

| Test Example | | 22 | 23 |
|---|---|---|---|
| (A) Sodium polyacrylate ^{*5} | | 0.05 | - |
| (A) Poly(2-acrylamido-2-methylpropane sulfonic acid) ^{*6} | | - | 0.05 |
| (B) Trehalose | | 0.5 | 0.5 |
| (C) Ethanol | | 10 | 10 |
| (C) Water | | Balance | Balance |
| (D) Hydrophobized (silicone resin-coated) titanium dioxide | | 11 | 11 |
| (E) Silica ^{*2} | | 2 | 2 |
| (F) Dimethicone | | 25 | 25 |
| (F) Octocrylene | | 3 | 3 |
| (F) Ethylhexyl methoxycinnamate | | 7 | 7 |
| Disteardimonium hectorite | | 0.5 | 0.5 |
| PEG-9 polydimethylsiloxyethyl dimethicone | | 3 | 3 |
| Total | | 100 | 100 |
| Evaluation | Moistness | A | A |
| | Without squeaky feeling | A | A |
| | Lightness of spreadability | A | A |
| *5: Weight-average molecular weight: 4,000,000 to 5,000,000 | | | |
| *6: Weight-average molecular weight: 4,000,000 to 5,000,000 | | | |

The water-in-oil composition to the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

Some or all of the foregoing embodiments may be described as in the following additional features, although not limited thereto. The various additional features may be employed in combination with the claim(s) in the Scope of Claims.

### [Additional Feature 1]

A method of using a water-in-oil composition, comprising using the water-in-oil composition of the present disclosure as an external-use skin preparation.

### [Additional Feature 2]

A method of using a water-in-oil composition, comprising using the water-in-oil composition of the present disclosure as a sun-screen cosmetics.

### Industrial Applicability

The water-in-oil composition of the present disclosure is applicable, for example, to cosmetics, cleansers, etc., applicable to the skin. The water-in-oil composition of the present disclosure is applicable, for example, to base makeup, veil makeup, makeup cosmetics, antiperspirants, deodorants, sun-screen cosmetics, skin-care agents, cleansers, etc.

## Claims

1. A water-in-oil composition comprising:
(A) from 0.02% by mass to 0.25% by mass of a water-soluble polymer;
(B) from 0.2% by mass to 2.5% by mass of at least one component selected from the group consisting of sugars and sugar alcohols, the component being in a solid form under atmospheric pressure at 25°C;
(C) water;
(D) a first powder having a hydrophobic particle surface;
(E) a second powder having a hydrophilic particle surface; and
(F) an oily component, wherein:
the component (A) is at least one selected from the group consisting of polyacrylic acid, poly(2-acrylamido-2-methylpropane sulfonic acid), and salts thereof; and
the component (E) has a linseed oil absorption amount at 25°C of 170 ml or less per 100 g.

2. The water-in-oil composition according to claim 1, wherein a content of the component (D) is from 2% by mass to 20% by mass relative to the mass of the composition.

3. The water-in-oil composition according to claim 1 or 2, wherein a content of the component (E) is from 1% by mass to 5% by mass relative to the mass of the composition.

4. The water-in-oil composition according to any one of claims 1 to 3, wherein the component (B) is at least one selected from the group consisting of trehalose, xylitol, and erythritol.

5. The water-in-oil composition according to any one of claims 1 to 4, wherein the component (D) is at least one selected from the group consisting of hydrophobized titanium dioxide and hydrophobized zinc oxide.

6. The water-in-oil composition according to any one of claims 1 to 5, wherein the component (E) is at least one selected from the group consisting of silica, corn starch, and cellulose.

7. The water-in-oil composition according to any one of claims 1 to 6, wherein the component (F) contains a liquid-state oily component and a UV absorber.

8. The water-in-oil composition according to any one of claims 1 to 7, wherein the component (A) has a weight-average molecular weight of from 500,000 to 8,000,000.

9. The water-in-oil composition according to any one of claims 1 to 8, wherein a content of a resinous powder having an average particle size of from 1 µm to 20 µm is 0.1% by mass or less relative to the mass of the composition.

10. The water-in-oil composition according to any one of claims 1 to 9, wherein the water-in-oil composition is for use in an external-use skin preparation.
